# EUROPEAN PATENT APPLICATION

(11) **EP 2 851 044 A1**
(43) Date of publication of application: **25.03.2015**
(21) Application number: 13185469.7
(22) Date of filing: 22.09.2013
(51) Int. Cl.: A61F 13/02

(54) **Wound dressing and method for manufacturing a wound dressing**

(71) Applicant: Absorbest AB, 590 39 Kisa (SE)
(72) Inventor: Rovaniemi, Rolf, 590 46 Rimforsa (SE)
(74) Representative: WSL Patentanwälte Partnerschaft mbB

(57) **Abstract**

The present invention relates to a wound dressing comprising an absorbent core, a facing layer, and a backing layer, wherein the absorbent core is located between the facing layer and the backing layer. Wound dressings are known, which may be designed such that they are self-adhesive to a patient's wound. When compared to the prior art it is an object of the present invention to provide a wound dressing causing less pain for a patient due to the expansion of the absorbent core. In order to solve this problem a wound dressing is suggested comprising an absorbent core, a facing layer, and a backing layer, wherein the absorbent core is located between the facing layer and the backing layer, wherein the wound dressing further comprises an adhesive being arranged and located for attaching the wound dressing to a patient's skin such that the facing layer faces the skin.

## Description

The present invention relates to a wound dressing comprising an absorbent core, a facing layer, and a backing layer, wherein the absorbent core is located between the facing layer and the backing layer.

Furthermore the present invention relates to a method for manufacturing a wound dressing comprising the following steps: Providing an absorbent core, providing a facing layer, providing a backing layer and locating the absorbent core between the facing layer and the backing layer.

Wound dressings containing superabsorbent polymers in particular for application on heavily secreting wounds have been described in various forms in the prior art. For example the book "More than superabsorbent polymer technology" edited by Frederic L. Buchholz and Andrew T. Graham, published in 1998 by John Wiley & Sons, Inc., ISBN 0-471-19411-5 on page 251 discloses the usage of superabsorbent polymers for the design of effective wound dressings.

WO 03/094813 describes a wound dressing comprising a facing layer and the backing layer forming a pouch, in which an absorbent core consisting of a non-woven material comprising superabsorbent polymers dispersed therein is located.

German patent DE 10 2007 019 622 B4 furthermore describes a wound dressing, which may be designed such that it is self-adhesive to a patient's wound. In order to provide this functionality a fluid-absorbent unit comprising a shell and an absorbent core is laminated with its backing onto a covering element extending beyond the edges of the fluid-absorbent unit, such that sections of the covering element may be fixed to the healthy skin surrounding a patient's wound. When absorbing exudate, the absorbent unit will increase in volume which is accommodated for by the covering element being continuously expandable. However, the wound dressing as suggested in DE 10 2007 019 622 D4 due to the properties of the covering element is a so-called occlusive bandage.

When compared to the prior art it is an object of the present invention to provide a wound dressing causing less pain for a patient due to the expansion of the absorbent core.

It is a further object to provide a wound dressing avoiding imprints on the healthy skin of the patient.

Furthermore it may be an object to provide a softer product enhancing the wearing comfort of the wound dressing.

From a production point of view it may be an object to provide a wound dressing allowing production at reduced cost.

At least one of the above objects is solved by a wound dressing comprising an absorbent core, a facing layer, and a backing layer, wherein the absorbent core is located between the facing layer and the backing layer, wherein the wound dressing further comprises an adhesive being arranged and located for attaching the wound dressing to a patient's skin such that the facing layer faces the skin.

In an embodiment the absorbent core may be any structure comprising cellulose fluff, air-laid cellulose, tissue paper, a non-woven, a textile fabric, a foam, an alginate, ALT or a hydrocolloid. In particular such a structure of the absorbent core in an embodiment may be used as a carrier layer to accommodate or carry an absorbent substance, in particular a superabsorbent substance.

The absorbent core of the wound dressing in an embodiment may be any structure comprising a superabsorbent substance. Superabsorbent substances in the sense of the present application are materials being able to absorb and retain large volumes of water in aqueous solutions. Superabsorbent substances falling into this category are for example modified starch, polymerized polyvinyl alcohol (PVA) and polyethylene oxide (PEO) which are all hydrophilic and have a high affinity to water. When chemically or physically cross-linked, these polymers are water-swellable but not water-soluble. The aforementioned superabsorbent substances have been known for a long time.

In a particular embodiment of the present invention the superabsorbent substance is a superabsorbent polymer (SAP), in particular in the form of (granular) particles or fibers. In an embodiment such a SAP is made from polymerization of acrylic acids blended with sodium hydroxide in the presence of an initiated form poly-acrylic acid sodium salt (sometimes referred to a sodium poly-acrylate).

In a further embodiment, the absorbent core containing SAP comprises a carrier layer, wherein the superabsorbent polymer is dispersed in the carrier layer. In an embodiment the carrier layer in particularl may comprise a material selected of a group consisting of tissue paper, a spunlaced polymer, a non-woven fabric or cellulose.

In an embodiment the absorbent core containing SAP comprises at least two carrier layers, which together with the SAP form the absorbent core. For manufacturing the SAP is dispersed on the first layer, then the second layer is put on top and the two layers are consolidated providing a matrix carrying the SAP between the two layers.

In an embodiment, the absorbent core comprises a carrier layer made of a spunlaced polymer as a non-woven fabric and a granular or fibrous SAP. For manufacturing the SAP in a first step is, preferably uniformly, dispersed on a first sheet or layer of the spunlaced nonwoven. In a second step a second sheet or layer of the spunlaced nonwoven is put onto the first sheet, such that the SAP is located between the two sheets or layers. Then the SAP is integrated in both layers by applying pressure to this sandwich structure provided. By applying pressure the two layers of spunlaced polymer are consolidated and the SAP to some extent fills up voids in the spunlaced material. The laminate formed this way is soft and looks like a single uniform layer of material.

A non-woven fabric in the sense of the present application is a material made of at least one layer of fibers which have been formed to a web and consolidated in a next step. In particular, consolidation of the non-woven fabric may be achieved by friction and/or cohesion and/or adhesion, for example by needling, felting, spun-lacing, melting or heat embossing.

If compared to tissue paper a material will be considered a non-woven fabric in the sense of the present application once more than 50 % of the mass of its fiber components consist of fibers having a ratio of their length to their diameter of more than 300. Alternatively the material will be considered a non-woven fabric in the sense of the present application if this condition is not ful-filled, but if more than 30 % of the mass of its fiber components consist of fibers having a ratio of their length to their diameter of more than 300 and its density is lower than 0.4 g / cm³. This is deemed to be equal to EM 29 092.

While an absorbent core as described above may be advantageous, it is not excluded to design absorbent cores using different material combinations.

An absorbent core, in particular an absorbent core having a superabsorbent substance which in the following text may also be denoted as a superabsorbent core, extracts and stores liquid exudates from a wound, to which the wound dressing is applied to.

In order to avoid direct contact between the absorbent core and the wound surface, the wound dressing further comprises a facing layer.

In order to avoid direct contact between the absorbent core and e.g. a patient's clothing, the wound dressing further comprises a backing layer.

In an embodiment, the facing layer comprises a material selected of a group consisting of a non-woven fabric, e.g. containing polyethylene (PE), polyethylene terephthalate (PET), polypropylene (PP), polyamide or polytetrafluoroethylene (PTFE), a perforated sheet, e.g. containing polyethylene (PE), polyethylene terephthalate (PET), polypropylene (PP), poly-amide or polytetrafluoroethylene (PTFE), a perforated sheet laminated on a non-woven fabric, e.g. containing polyethylene (PE), polyethylene terephthalate (PET), polypropylene (PP), polyamide or polytetrafluoroethylene (PTFE), a fine net or screen, e.g. containing polyethylene (PE), polyethylene terephthalate (PET), polypropylene (PP), polyamide or polytetrafluoroethylene (PTFE), a perforated foam or sheet comprising polyurethane, a perforated material based on silicone or a foam with open cells based on polyurethane or silicone or a combination thereof.

In an embodiment, wherein the facing layer comprises a perforated sheet or film, wherein the perforations form a three-dimensional structure in order to reduce the sticking surface between the facing layer and the wound surface.

In an embodiment, the facing layer comprises a non-woven fabric consisting of synthetic and/or cellulose fibers. In an embodiment the fibers of a non-woven fabric forming a facing layer are reoriented such that they predominantly extend in a direction perpendicular to the extension of the facing layer. Such a reorientation of the fibers in the non-woven fabric is achieved by orienting the fibers during the fabrication process, in particular during spun-lacing, needling or electrostatic processing.

In an embodiment of the invention the facing layer comprises a density in a range from 0.1 g / cm³ to 0.6 g / cm³.

In a further embodiment, the facing layer made of a nonwoven in its unwetted state comprises an area weight (also denoted as the gram weight, basis weight or grammage) in a range from 8 gsm (g / m²) to 50 gsm (g / m²), preferably in a range from 12 gsm (g / m²) + 30 gsm (g / m²).

It is further useful if in an embodiment the facing layer comprises a hydrophobic or hydrophilic surface. This is in particular applicable once the facing layer is brought into direct contact with the wound. Therefore the facing layer in an embodiment may be coated by non-sticking material, e.g. silicone. In an embodiment the non-sticking material on the facing layer may be structured to form a pattern on the facing layer.

While it may be that the facing layer is brought into direct contact with the wound (it may then be denoted the contact layer), there may be an embodiment, wherein the wound dressing comprises one or more further layers between the facing layer and the wound. Depending on its functionality in the wound dressing, a person skilled in the art will choose the material of the facing layer in order to fulfil such functionality.

In an embodiment of the present invention, the backing layer serves as a clothing protection. In an embodiment, the backing layer thus is advantageously made of a breathable, non-woven fabric or a breathable film enabling breathing of the wound, but preventing wound exudates from exiting the wound dressing an contaminating a patient's clothing. In a further embodiment the backing layer, in particular when made of a non-woven fabric, is hydrophobic.

In an embodiment of the invention the backing layer is a hydrophobic nonwoven based on polypropylene with a hydro head in a range from 40 cm H₂O to 120 cm H₂O preferably in a range from 50 cm H₂O to 80 cm H₂O. A backing fulfilling this requirement on the one hand provides a good protection of a patient's clothing while on the other hand avoiding bacteria to enter into the wound dressing. The hydro head in the sense of the present application of height of a vertical water column standing on and above the surface of the material and which the material can stand against before the water passes through the material to the other side. The numbers given for the hydro head are measured according to ISO 811:1981.

An adhesive in the sense of the present application may be any adhesive substance, which is suitable in order to fix a wound dressing removably to a patient's skin, preferably to the healthy parts of the skin surrounding a wound. Such an adhesive may for example be an adhesive comprising acrylate or silicone.

It is assumed that in an embodiment the facing layer and/or the backing layer and/or the absorbent core is a laminar structure whose thickness is small compared to its length and width. As such the extension of these layers denotes their laminar extension. A direction parallel to the extension of the layers is essentially parallel to the plane defined by the width and the length of the layers. A direction perpendicular to the extension of these layers essentially denotes the thickness of the respective layer.

In an embodiment, the facing layer and the backing layer are integrally formed from a single sheet of material. However, in some embodiments the facing layer and the backing layer are two distinct and separate layers, which enables to design their properties and functionalities individually as needed.

In an embodiment the different layers of the wound dressing are stacked on top of each other and preferably are connected to each other, e.g. by gluing or heat-embossing. The assembly of the absorbent core, a facing layer and a backing layer do form a structure which is frequently denoted as a wound pad or an absorbent unit in the field.

According to an embodiment of the present invention, a shell/cover enclosing the absorbent core may be formed by the facing layer and the backing layer, wherein the facing layer and the backing layer are joint together such that the facing layer and the backing layer form a pouch. It is evident, that the absorbent core when it is located in the pouch, is surrounded by the facing layer and the backing layer.

In order to form a pouch, the facing layer and the backing layer may be joint together by a seam, e.g. by gluing, heat-sealing, e.g. ultrasonic sealing, or embossing.

In an embodiment the absorbent core has a top surface and a bottom surface, wherein the facing layer is folded such that it covers the entire bottom surface of the absorbent core and such that it comprises at least two sections covering parts of the top surface of the absorbent core, wherein the seam is at least partly formed between the sections of the facing layer covering parts of the top surface of the absorbent core and the backing layer.

Typically the facing layer and the backing layer are joint together by a seam which lies outside the area covered by the absorbent core and thus at least partly encloses or surrounds the absorbent core. In the areas, where facing layer and the backing layer are connected by the seam, the wound dressing may tend to be less flexible than the actual separated layers forming the cover without the seam would be. It has been observed that the area of the seam for these embodiments may lead to irritation or redness of the patient's skin surrounding the actual wound. In order to reduce skin irritations by the seam in an embodiment of the present invention it is suggested to transfer the seam or the seams such that they predominantly or exclusively are located above the absorbent core when viewed from the wound.

According to an embodiment of the present invention, this transfer of the seam is achieved by designing the facing layer, i.e. the layer, which in use of the wound dressing is facing towards the wound or is located closely to the wound, such that it is folded around an edge of the absorbent core. The facing layer then covers the entire bottom surface of the absorbent core and comprises at least two sections covering part of the top surface of the absorbent core. When subsequently the seam or the seams are at least partly formed between those sections of the facing layer covering parts of the top surface of the absorbent core and the backing layer, the majority of the extension of the seam may not get into direct contact with the wound, but is transferred to a position above the absorbent core when viewed from the wound.

There may be embodiments, wherein some parts of the seam are still formed next to the absorbent core or extending beyond the absorbent core. There may also be embodiments, wherein the entire seam is exclusively formed between sections of the facing layer covering parts of the top surface of the absorbent core and the backing layer.

In an embodiment the adhesive may be directly applied to the facing layer, wherein optionally the adhesive only partly covers the facing layer.

In an alternative embodiment of the present invention the wound dressing comprises a carrier, in particular a film or a sheet, made of a polymer with an adhesive. The carrier with the adhesive in an embodiment is arranged on the facing layer. The adhesive in an embodiment is applied to the carrier such that they form an integrated structure. In this integrated structure in an embodiment the carrier and the adhesive have the same lateral dimensions, as it is for example the case with a sticky tape. It is evident that when in use the adhesive on the carrier faces the patient's skin in order to fix the wound dressing to the skin.

In an embodiment the carrier comprises polyurethane or a thermoplastic elastomer. In particular combinations of a carrier comprising polyurethane and an adhesive comprising acrylate or a carrier comprising a thermoplastic elastomer and an adhesive comprising silicone have proven to be advantageous.

In an embodiment the carrier film with the adhesive only partly covers the facing layer exposing the facing layer such that exudate from the wound may enter through the facing layer and may be transported to the absorbent core.

This design provides numerous advantages. Under a first aspect a simplified production line can be used for manufacturing, leading to an increase in productivity and a decrease in costs. Under a second aspect when absorbing liquid, the absorbent core will extend its dimensions predominantly in a direction perpendicular to its lateral extension, i.e. it will get thicker. An embodiment, wherein the carrier film and the adhesive only partly cover the facing layer, will accommodate for such a swelling of the absorbent core when absorbing liquid.

In a further embodiment the carrier with the adhesive comprises a cutout exposing the facing layer for accommodating an expansion of the wound dressing when the absorbent core during use of the wound dressing absorbs exudate from a wound.

Preferably such a cutout is located centrally such that the cutout is right at a position where the absorbent core is located and absorbs most of the exudate from a wound. In particular it is advantageous if the cutout of the carrier film is located at the position, where the wound is located when the wound dressing is applied to a patient's skin.

In a further embodiment the carrier film with the adhesive forms a ring-like structure, such that in a central part of the ring the facing layer is exposed for accommodating an expansion of the wound dressing. A ring-like structure in the sense of the present application is not only a ring having a circular shape, but could in principle have all types of shapes as long as it comprises an outer and an inner edge being circumferential, i.e. closed.

In an alternative embodiment the adhesive is directly applied to the facing layer, wherein the adhesive only partly covers the facing layer.

In a further embodiment an adhesive is arranged on the facing layer forming a ring-like structure, such that in the central part of this ring-like structure the facing layer is exposed to be brought into contact with the patient's wound.

In a further embodiment of the present invention, the wound dressing either alternatively or additionally comprises a carrier with an adhesive being applied to the backing layer, wherein the carrier with the adhesive extends beyond the edges of the backing layer, and wherein the carrier with the adhesive comprises a cutout placed on the backing layer exposing the backing layer for accommodating an expansion of the absorbent core during use of the wound dressing. In an embodiment the adhesive directly applied to the backing layer comprises a material selected from a group consisting of acrylate, silicone, synthetic rubber or thermoplastic rubber or a combination thereof. An adhesive, which may be suitable for application directly onto the facing layer is commercially available from Henkel of Düsseldorf, Germany under the trade names Sanicare and Dispomelt or from Nolax, Switzerland.

At least one of the above objects is also solved by a method for manufacturing a wound dressing comprising the steps: providing an absorbent core, providing a facing layer, providing a backing layer, locating the absorbent core between the facing layer and the backing layer, and further applying an adhesive to the wound dressing for attaching the wound dressing to a patient's skin such that the facing layer faces the skin.

As far as aspects of the present invention have been described above regarding the wound dressing, they also apply to a process for manufacturing such wound dressing and vice versa. Further advantages, features and applications of the present invention will become apparent from the following description of embodiments and the corresponding figures attached.
Figure 1 shows a schematic top view onto the facing side of a wound dressing according to an embodiment of the present invention.
Figure 2 shows a schematic top view onto the wound dressing of figure 1 from a backing side.
Figure 3 shows a schematic cross-sectional view along line A-A of figure 2.
Figure 4 shows a schematic cross-sectional view of a wound dressing according to figures 1 to 3 along line B-B of figure 2.
Figure 5 shows a schematic top view of a second embodiment according to the present invention from a facing side.
Figure 6 shows a schematic top view of the wound dressing of figure 5 from a backing side.
Figure 7 shows a schematic cross-sectional view of a wound dressing according to figures 5 and 6 along line A-A of figure 6.
Figure 8 shows a schematic cross-sectional view of the wound dressing according to figures 5 to 7 along line B-B of figure 6.
Figure 9 shows a schematic top view onto the facing side of a wound dressing according to yet another embodiment of the present invention.
Figure 10 shows a schematic cross-sectional view of the wound dressing of figure 9 along line A-A.

In the figures identical elements have been denoted by identical reference numbers. The figures only represent schematic views and are not to scale.

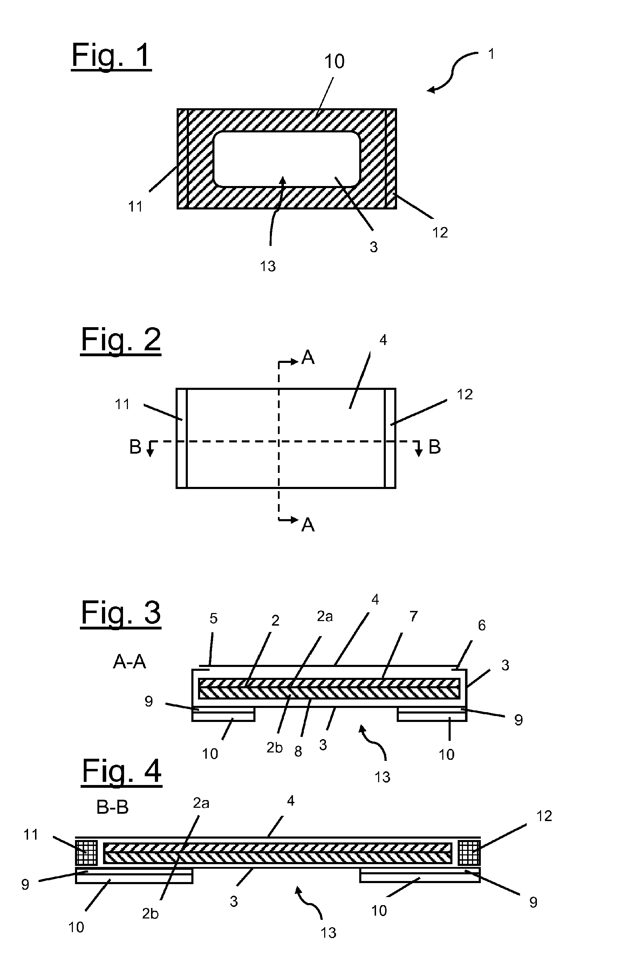

According to figures 1 to 4 a first embodiment of a wound dressing comprising features of the invention is schematically depicted.

The wound dressing 1 consists of a layered structure which is best understood when considered with reference to the cross-sectional drawings of figures 3 and 4.

The wound dressing 1 comprises an absorbent core 2, which in this particular embodiment has two sub-layers 2a, 2b made of tissue paper with a superabsorbent polymer in the form of granular particles dispersed between the two sub-layers 2a, 2b. The two sub-layers together form the carrier layer of the absorbent core. In order to manufacture the absorbent core 2 the superabsorbent particles are dispersed on a first sub-layer 2b thereof and then the second sub-layer 2a is consolidated on top of the first sub-layer 2b providing a fixation of the superabsorbent particles.

In order not to come into direct contact with the wound on the one side and e.g. a clothing of the patient on the other side, the absorbent core 2 is wrapped or enveloped in a pouch formed by a facing layer 3 and a backing layer 4.

The facing layer 3 in this particular embodiment is made of a white hydrophilic non-woven fabric consisting of polypropylene fibers in order to provide a good transport of exudate from a wound to the absorbent core 2.

As an example the backing layer serves as a clothing protection and is made of a green breathable, hydrophobic non-woven fabric based on polypropylene. The non-woven has a hydro head of 50 cm/H₂O. This backing layer allows breathing of the wound while simultaneously preventing wound exudates from exiting the wound dressing and contaminating a patient's clothing.

The facing layer 3 and the backing layer 4 have been joint together by two seams 5, 6 extending above the absorbent core. In order to do so, the facing layer 3 has been folded in parallel to its long edges such that it also extends above the absorbent core 2 and it partly covers the top surface 7 of the absorbent core 2. Furthermore the facing layer 3 entirely covers the bottom surface 8 of the absorbent core 2.

A location of the seams in an area above the absorbent core 2, i.e. in a position where the facing layer 3 and the backing layer 4 overlap above the absorbent core 2, has the advantage that no reddening due to stiff edges along the long side of the wound dressing occur. When considered in a cross-section along the line A-A of figure 2 the wound dressing 1 thus has a tubular structure as shown in figure 3.

The cross-sectional view of figure 4 taken along line B-B of figure 2 shows that the facing layer 3 and the backing layer 4 along the short edges of the wound dressing 1 are conventionally sealed by glue lines 11, 12 which extend next to the absorbent core 2.

Furthermore the wound dressing 1 comprises a carrier 9 provided on the facing layer 3 in order to support an adhesive 10 for fixing the wound dressing 1 at the healthy skin surrounding a patient's wound.

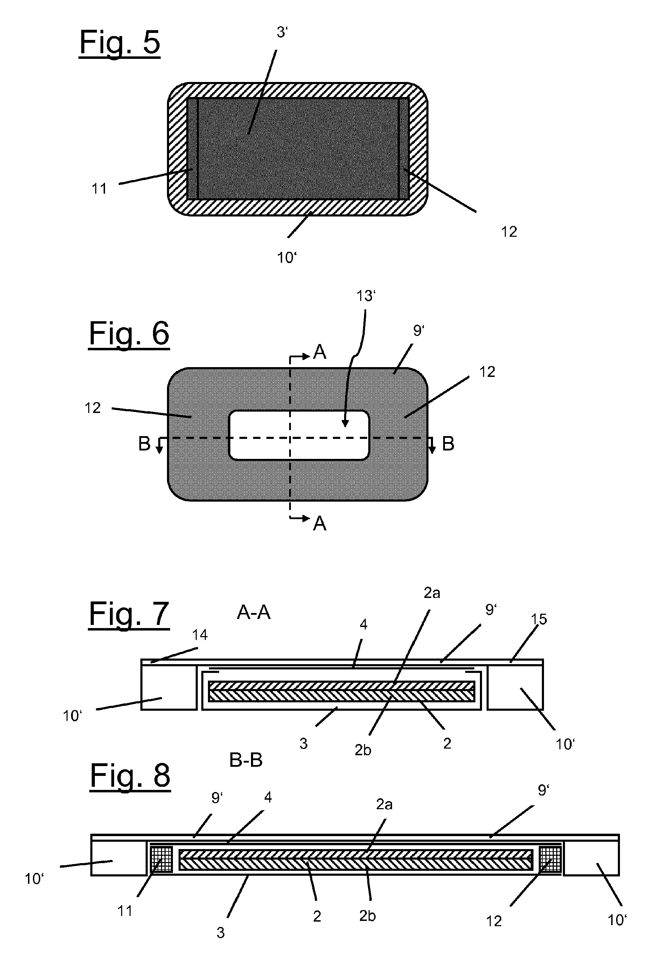

In the present embodiment the carrier 9 serving as a carrier for the adhesive 10 is a polyurethane carrier with an acrylate adhesive 10 on top. The carrier 9 and the adhesive together form an integrated structure similar to e.g. a conventional sticky tape. Thus after cutting the carrier and the adhesive do have the same lateral extension or dimensions. The same applies to the carrier 9' and adhesive 10' according to the embodiment of figures 5 to 8.

While the carrier 9 and the adhesive 10 extend up to the outer edges of the shell formed by the facing layer 3 and the backing layer 4, the carrier 9 and thus the adhesive 10 applied to the carrier comprises a cutout 13 arranged in a central part of the wound dressing below the absorbent core 2. In this cutout 13 which is depicted most clearly in figure 1, the facing layer 3 is exposed such that it may get into contact with the patient's wound. An exudate from the wound can thus be transported through the facing layer 3 into the absorbent core 2.

By providing the cutout section 13 in the carrier 9 and the adhesive 10, one gains a further degree of freedom when choosing the material of the adhesive 10 as well as for the carrier 9. The carrier 9 and the adhesive 10 e.g. have a MVTR of 1300 qsm/m2/day (measured with the inverted bottle method).

Furthermore the cutout section 13 allows for an expansion of the absorbent core 2 when exudate from the wound is absorbed in the absorbent core 2. This absorption will lead to a swelling predominantly in a direction perpendicular to the lateral extension of the facing layer, i.e. an increase in the thickness of the absorbent core 2. By the cutout 13, the carrier 9 and the adhesive 10 need not account for this swelling but the increase in volume of the absorbent core 2 can occur without any resistance in the area of the cutout 13.

Considering the representation of figure 1, the carrier 9 as well as the adhesive 10 applied to the carrier 9 form a ring-like structure in the sense of the present application, while the cutout is provided in a central area of the wound dressing 1.

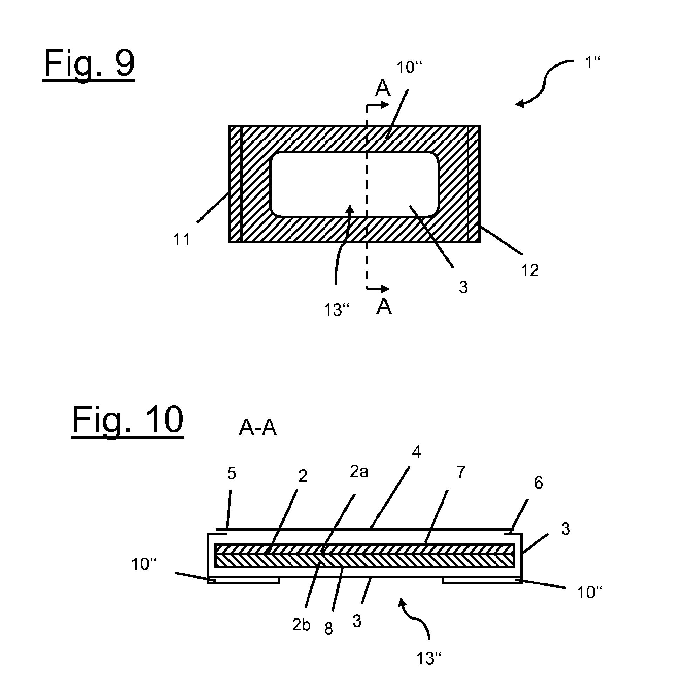

In a second embodiment depicted in figure 9 and 10 the design of the wound dressing 1"' is identical to the embodiment described before with reference to figures 1 to 4, except that it lacks a carrier for the adhesive, but the adhesive 10" is directly applied to the facing layer 3 of the wound dressing. This is well visible from the cross-sectional view of figure 10.

In order to be able to apply the adhesive 10" directly to the facing layer 3 made of a white hydrophilic non-woven fabric consisting of polypropylene fibers an adhesive based on silicone is used as the adhesive 10".

A further embodiment of a wound dressing 1' depicted in figures 5 to 8 has an identical design when compared to the wound dressings 1, 1" of figures 1 to 4 and 9, 10 when it comes to the constructive features of the wound pad consisting of a facing layer 3, a backing layer 4 and an absorbent core 2.

However, instead of having a carrier 9 with an adhesive 10 applied onto the facing layer 3, the carrier 9' with the adhesive 10' according to the embodiment of figures 5 to 8 is applied on top of the backing layer 4. The carrier 9' with the adhesive 10' extends in size beyond the edges of the cover or shell formed by the facing layer 3 and the backing layer 4. The adhesive 10' is also applied to those sections 14, 15 extending beyond the edges of the wound pad is exposed in order to allow for a fixing of the wound dressing 1' to a patient's skin.

When viewed in a top view onto the backing layer 4 as depicted in figure 6, it becomes apparent, that the carrier 9' and the adhesive 10' as before comprise a cutout section 13' which in this embodiment exposes a central part of the backing layer 4. Again this cutout section 13' of the carrier 9' and the adhesive 10' has to accommodate for a swelling, i.e. an increase in volume, of the absorbent core 2.

For purposes of original disclosure it is pointed out that all features which are apparent for a person skilled in the art from the present description, the figures and the claims, even if they have only been described with further features, could be combined on their own or together with all the combinations of the features disclosed herein, if not excluded explicitly or technically impossible. A comprehensive explicit description of all possible combinations of features is only omitted in order to provide readability of the description.

### Reference List

- 1, 1', 1": Wound dressing
- 2: Absorbent core
- 2a, 2b: Sub-layers as a carrier layer for SAP of the absorbent core
- 3: Facing layer
- 4: Backing layer
- 5, 6: Seam
- 7: Top surface of the absorbent core 2
- 8: Bottom surface of the absorbent core 2
- 9, 9': Carrier
- 10, 10', 10": Adhesive
- 11, 12: Glue line
- 13, 13', 13": Cutout
- 14: Section of the carrier film extending beyond the cover
- 15: Section of the carrier film extending beyond the cover

## Claims

1. A wound dressing (1, 1', 1 ") comprising
an absorbent core (2),
a facing layer (3), and
a backing layer (4),
wherein the absorbent core (2) is located between the facing layer (3) and the backing layer (4), **characterised in that** the wound dressing (1, 1', 1 ") further comprises an adhesive (10, 10', 10") being arranged and located for attaching the wound dressing (1, 1', 1") to a patient's skin such that the facing layer (3) faces the skin.

2. A wound dressing (1) according to claim 1, **characterised in that** the wound dressing (1) further comprises a carrier (9) being arranged on the facing layer (3), wherein the adhesive (10) is applied to the carrier (9) such that the carrier (9) forms a carrier for the adhesive (10), and wherein the carrier (9) only partly covers the facing layer (3) exposing some sections of the facing layer (3).

3. A wound dressing (1) according to claim 2, **characterised in that** the carrier (9) with the adhesive (10) comprises a cut-out (13) exposing the facing layer (3) for accommodating an expansion of the wound dressing (1) when the absorbent core (2) during use of the wound dressing (1) absorbs exudate from a wound.

4. A wound dressing (1) according to claim 2 or claim 3, **characterised in that** the carrier (9) with the adhesive (10) forms a ring-like structure, such that in a central part of the ring-like structure the facing layer (3) is exposed for accommodating an expansion of the wound dressing (1) when the absorbent core (2) during use of the wound dressing (1) absorbs exudate from a wound.

5. A wound dressing (1, 1') according to any of the previous claims, **characterised in that** the carrier (9, 9') comprises polyurethane and the adhesive (10, 10') comprises acrylate.

6. A wound dressing (1, 1') according to any the previous claims, **characterised in that** the carrier (9, 9') comprises a thermoplastic elastomer and the adhesive (10, 10') comprises silicone.

7. A wound dressing (1, 1', 1 ") according to any of the previous claims, **characterised in that** the facing layer (3) and the backing layer (4) are joint together by a seam (5, 6) to form a pouch, wherein the absorbent core (2) is located in the pouch.

8. A wound dressing (1, 1', 1 ") according to claim 7, **characterised in that** the absorbent core (2) has a top surface (7) and a bottom surface (8), wherein the facing layer (3) is folded such that it covers the entire bottom surface (8) of the absorbent core (2) and such that it comprises at least two sections covering parts of the top surface (7) of the absorbent core (2), wherein the seam (5, 6) is at least partly formed between the sections of the facing layer (3) covering parts of the top surface (7) of the absorbent core (2) and the backing layer (4).

9. A wound dressing (1, 1', 1 ") according to any of the previous claims, **characterised in that** the absorbent core (2) comprises a carrier layer (2a, 2b) and a super absorbent polymer dispersed in the carrier layer (2a, 2b).

10. A wound dressing (1, 1', 1 ") according to claim 9, **characterised in that** the carrier layer (2a, 2b) comprises tissue paper.

11. A wound dressing (1, 1', 1 ") according to any of the previous claims, **characterised in that** the carrier layer (2a, 2b) comprises a spun-laced polymer.

12. A wound dressing (1 ") according to claim 1, **characterised in that** the adhesive (10") is directly applied to the facing layer (3), wherein the adhesive (10") only partly covers the facing layer (3).

13. A wound dressing (1 ") according claim 12, **characterised in that** the adhesive (10") is arranged on the facing layer (3) forming a ring-like structure, such that in a central part of this ring-like structure the facing layer (3) is exposed to be brought into contact with a patient's wound.

14. A wound dressing (1') according to any of the previous claims, **characterised in that** the wound dressing (1') further comprises a carrier (9') with the adhesive applied to a surface of the carrier film (9'), wherein the carrier with the adhesive (10') is applied to the backing layer (4), wherein the carrier (9') with the adhesive (10') extends beyond the edges of the backing layer (4), and wherein the carrier (9') comprises a cut-out (13') exposing the facing layer (3) for accommodating an expansion of the absorbent core (2) when the absorbent core (2) during use of the wound dressing (1') absorbs exudate from a wound.

15. A method for manufacturing a wound dressing (1, 1', 1 ") comprising the steps:
providing an absorbent core (2),
providing a facing layer (3),
providing a backing layer (4), and
locating the absorbent core (2) between the facing layer (3) and the backing layer (4),
**characterized in that**
it further comprises the step of applying an adhesive (10, 10', 10") to the wound dressing (1, 1', 1 ") for attaching the wound dressing (1, 1', 1 ") to a patient's skin such that the facing layer (3) faces the skin.
